# EUROPÄISCHE PATENTANMELDUNG

(11) **EP 2 606 919 A1**
(43) Veröffentlichungstag der Anmeldung: **26.06.2013**
(21) Anmeldenummer: 11075272.2
(22) Anmeldetag: 22.12.2011
(51) Int. Cl.: A61M 1/10, A61M 1/12, A61M 25/06

(54) **Schleuseneinrichtung zum Einführen eines Katheters**

(71) Anmelder: ECP Entwicklungsgesellschaft mbH, 12247 Berlin (DE)
(72) Erfinder: Schumacher, Jörg, 14513 Teltow (DE); Bredenbreuker, Lars, 10781 Berlin (DE); Decke, Robert, 12247 Berlin (DE)
(74) Vertreter: Pfenning, Meinig & Partner GbR

(57) **Zusammenfassung**

Bei einer Schleuseneinrichtung zum Einführen eines Katheters in einen Patientenkörper mit einer ersten Schleuse (10, 11, 13, 21,21', 21", 41, 43) mit einem proximalen Ende und einem distalen Ende, wobei beim bestimmungsgemäßen Gebrauch das distale Ende der ersten Schleuse zur Anordnung im Patientenkörper und das proximale Ende der ersten Schleuse zur Anordnung außerhalb des Patientenkörpers vorgesehen ist und wobei die erste Schleuse einen schlauchförmigen Abschnitt (11, 21, 21', 21", 41) und ein an dessen proximalem Ende angeordnetes Schleusengehäuse (13, 43) mit einem Aufnahmekanal (46) für einen Katheter aufweist, ist gemäß der Erfindung vorgesehen, dass der schlauchförmige Abschnitt (11, 21, 21', 21", 41) in einer Klemmvorrichtung (45, 48, 50, 62, 63, 64) des Schleusengehäuses lösbar kraftschlüssig gehalten ist, um eine Kürzung des schlauchförmigen Abschnitts in einfacher Weise vornehmen zu können.

## Beschreibung

Die Erfindung liegt auf dem Gebiet der Mechanik und ist mit Vorteil in der Medizintechnik einsetzbar. Sie befasst sich mit einer Schleuseneinrichtung zum Einführen eines Katheters in einen Patientenkörper, wobei die Schleuse in den Patientenkörper hineinführt und ein proximales Ende der Schleuse aus diesem herausragt. Die Schleuse stellt ein Lumen bereit, durch das ein Katheter in den Patientenkörper eingeführt werden kann.

Derartige Schleuseneinrichtungen sind grundsätzlich bekannt. Sie werden zum Einführen verschiedenartiger Katheter, beispielsweise im minimalinvasiven medizinischen Bereich, angewendet. Eine derartige Schleuse kann beispielsweise zum Einführen einer Blutpumpe zur Herzunterstützung vorgesehen sein, wobei eine solche Einheit eine distale Pumpeneinheit sowie einen Hohlkatheter und eine durch den Hohlkatheter geführte Antriebswelle aufweisen kann. Solche Pumpen werden im miniaturisierten Bereich oft derart ausgestaltet, dass sie radial komprimierbar und im komprimierten Zustand mit dem Katheter zusammen durch ein Blutgefäß des Körpers einführbar sind. Am Einsatzort, beispielsweise in einem Blutgefäß oder in einer Herzkammer, kann die Pumpe dann expandiert werden. Im expandierten Zustand mit aufgestellten Förderelementen kann eine solche Pumpe dann die notwendige Pumpleistung entfalten.

Außer solchen komprimierbaren Herzpumpen sind auch andere Funktionselemente denkbar, die mittels einer erfindungsgemäßen Schleuse in einen Hohlraum eines Körpers eingebracht werden, wie beispielsweise Stents oder Fräsköpfe für die Beseitigung von Gefäßablagerungen.

Das Einführen solcher Funktionselemente und Katheter mittels einer Schleuse ist gegenüber einem direkten Einführen beträchtlich einfacher und auch mit geringeren medizinischen Risiken verbunden.

Die Schleuse selbst kann mittels der bekannten Seldinger-Technik beispielsweise in ein Gefäßsystem eingeführt werden. Zu diesem Zweck wird zunächst mittels einer Punktionsnadel eine Öffnung in ein Körpergefäß eingebracht, worauf ein Führungsdraht eingeschoben wird. Über den Führungsdraht wird dann gegebenenfalls ein Dilatator und darauf die Schleuse selbst eingeschoben. Danach kann der Führungsdraht entfernt werden, wenn er nicht für weitere Führungsaufgaben benötigt wird, und weitere Elemente können durch die Schleuse eingeschoben werden.

Ein entsprechendes Verfahren ist beispielsweise aus der WO 02/43791 A1 bekannt. Dort ist vorgesehen, eine Herzpumpe entlang eines Führungsdrahtes in den linken Herzventrikel eines Patienten vorzuschieben und eine Pumpeneinheit aus der Schleuse heraus durch das Gefäßsystem zum Ventrikel vorzuschieben.

Eine entsprechende Fluidpumpe, die für hohe Drehzahlen vorgesehen ist, um eine entsprechende Pumpleistung entfalten zu können, ist in der Bauform als Blutpumpe ebenfalls aus der WO 02/43791 A1, jedoch auch aus der EP 2047872 A1 bekannt. Aus der EP 2 047 872 A1 ist eine Pumpe ersichtlich, die eine distale Pumpeneinheit aufweist, an die sich ein proximaler Wellenhohlkatheter anschließt. Eine in dem Wellenkatheter verlaufende Antriebswelle wird mit einer Antriebseinheit zum Antrieb des Rotors der Pumpe verbunden.

Die Verwendung einer komfortablen Schleuse zum Einführen eines Katheters, insbesondere mit einer Antriebswelle, bringt den Vorteil, dass beim Einführen der Katheter und insbesondere eine Antriebswelle wenig mechanisch belastet wird. Dies ist insbesondere bei den hohen mechanischen Belastungen, denen eine Antriebswelle im Betrieb einer Blutpumpe ausgesetzt ist, vorteilhaft.

Für eine komfortable Verwendung einer entsprechenden Schleuse zum Einführen eines Katheters, insbesondere mit einer distalen Pumpeneinheit, ist es wünschenswert, die Schleuse mit dem Hohlkatheter möglichst weit bis in die Nähe des Einsatzortes vorschieben zu können und dann den Wellenkatheter bzw. die Pumpe aus der Schleuse auszubringen, um die Schleuse daraufhin wenigstens ein Stück weit zurückziehen zu können. Auf diese Weise wird die einzubringende Einheit eine möglichst kurze Strecke im Gefäßsystem bzw. entsprechenden Hohlräumen des Patientenkörpers außerhalb einer Schleuse bewegt, so dass die Belastung der Gefäßwandungen einerseits durch das Einführen des Fremdkörpers und die mechanische Belastung der einzuführenden Einheit andererseits weitgehend reduziert ist.

Auf der anderen Seite erfordert ein derartiges Vorgehen eine entsprechende Überlänge der Schleuse, die nach dem Vorschieben und nachfolgenden Zurückziehen üblicherweise ein Stück weit aus dem Patientenkörper hervorragt.

Der vorliegenden Erfindung liegt die Aufgabe zugrunde, eine entsprechende Schleuseneinrichtung vorteilhaft so zu gestalten, dass sie besonders einfach handhabbar ist.

Die Aufgabe wird mit den Merkmalen der Erfindung gemäß Patentanspruch 1 sowie mit einem Kathetersystem entsprechend Patentanspruch 11 und unter Verwendung eines Verfahrens gemäß Patentanspruch 12 gelöst.

Die Erfindung sieht vor, dass eine Schleuseneinrichtung zum Einführen eines Katheters in einen Patientenkörper eine erste Schleuse mit einem proximalen Ende und einem distalen Ende aufweist, wobei das distale Ende der ersten Schleuse beim bestimmungsgemäßen Gebrauch innerhalb des Patientenkörpers angeordnet ist, während das proximale Ende aus dem Patientenkörper herausragt. Die erste Schleuse weist zudem einen schlauchförmigen Abschnitt und ein an dessen proximalem Ende angeordnetes Schleusengehäuse mit einem Aufnahmekanal für einen strangförmigen Körper, insbesondere für einen Katheter, auf.

Dadurch, dass der schlauchförmige Abschnitt in einer Klemmvorrichtung des Schleusengehäuses lösbar kraftschlüssig gehalten ist, kann zunächst zum Einführen des Katheters in den Patientenkörper die Schleuseneinrichtung insgesamt ein Stück weit in den Patientenkörper hineingeschoben, darauf der Katheter extrahiert und dann die Schleuseneinrichtung aus dem Patientenkörper ein Stück weit wieder herausgezogen werden. Danach kann in besonders einfacher Weise der schlauchförmige Abschnitt aus der Klemmung gelöst, gekürzt und erneut geklemmt werden.

Hierdurch ist es besonders einfach, die Schleuseneinrichtung nach dem Gebrauch derart passend abzulängen, dass sie nicht weiter als notwendig aus dem Patientenkörper herausragt.

Eine vorteilhafte Ausgestaltung der Erfindung sieht vor, dass der schlauchförmige Abschnitt im gelösten Zustand der Klemmvorrichtung in das Schleusengehäuse hinein verschiebbar ist. Auf diese Weise kann der schlauchförmige Abschnitt entweder in dem Schleusengehäuse aufgenommen, innerhalb des Schleusengehäuses entfernt oder durch eine proximale Öffnung des Schleusengehäuses entfernt werden.

Besonders sinnvoll erscheint es, dass der schlauchförmige Abschnitt in direkter Verlängerung des Aufnahmekanals für den Katheter in dem Schleusengehäuse mündet. Auf der einen Seite ist durch diese Ausgestaltung das Einführen eines Katheters in den schlauchförmigen Abschnitt besonders einfach, indem der Katheter zunächst in das Schleusengehäuse eingeführt, in dessen Aufnahmekanal geführt und damit ohne weiteres zur Mündung des schlauchförmigen Abschnittes geleitet wird. Auf diese Weise kann sichergestellt werden, dass das Einführen des Katheters in das proximale Ende der Schleuseneinrichtung vereinfacht ist und zuverlässig gelingt. Andererseits kann durch diese Ausgestaltung das proximale Ende des schlauchförmigen Abschnittes der Schleuse in einfacher Weise in den Aufnahmekanal innerhalb des Schleusengehäuses hineingeschoben werden, um dort entweder aufgenommen zu werden oder entfernt zu werden.

Eine weitere vorteilhafte Ausgestaltung der Erfindung sieht vor, dass die Klemmvorrichtung einen elastisch verformbaren Klemmring aufweist, der den schlauchförmigen Abschnitt umgibt und der durch eine Manipulationsvorrichtung derart pressbar ist, dass er den schlauchförmigen Abschnitt radial einklemmt. Eine derartige Klemmvorrichtung ist mechanisch äußerst einfach aufgebaut, zuverlässig, platzsparend und einfach zu bedienen. Sie bewirkt eine kraftschlüssige Fixierung des schlauchförmigen Abschnitts, die in einfacher Weise auch wieder lösbar ist.

Es kann hierzu vorgesehen sein, dass der Klemmring durch axiale Druckwirkung radial verformbar ist. Er kann zu diesem Zweck entweder als Elastomerring ausgestaltet sein oder als geschlitzter Ring aus einem Kunststoff oder einem Metall bestehen.

Der Klemmring kann als Elastomerring beispielsweise mittels eines Druckstücks flachdrückbar sein, wobei er radial nach innen und außen expandiert, wodurch der Innendurchmesser des Rings verringert wird. Handelt es sich bei dem Klemmring um einen geschlitzten Kunststoff- oder Metallring, so kann dieser beispielsweise unter Einwirkung eines Keilkörpers an seiner radialen Außenseite radial nach innen komprimiert werden. Beispielsweise kann zu diesem Zweck ein axial in Bezug auf das schlauchförmige Element bewegter, im Querschnitt keilförmiger Ring verwendet werden. Der Klemmring kann ebenfalls im Querschnitt konisch gestaltet sein. In einer bevorzugten Ausführung handelt es sich um einen Klemmring, der gleichzeitig eine fluiddichte Verbindung zwischen dem Schleusengehäuse und dem schlauchförmigen Element sicherstellt, wie es beispielsweise in Form des erwähnten Elastomerrings realisiert werden kann. Die Dichtheit kann aber, beispielsweise bei Verwendung eines geschlitzten Kunststoff- oder Metallrings, auch durch ein zusätzliches Dichtelement realisiert werden.

Die Klemmvorrichtung kann beispielsweise zur axialen Pressung eines Klemmrings eine Schraubvorrichtung aufweisen. Mittels der Schraubvorrichtung kann dann beispielsweise ein Druckstück gegen den Klemmring in axialer Richtung gedrückt werden.

Zur Erleichterung der Abtrennbarkeit von Teilen des schlauchförmigen Abschnitts zu dessen Kürzung kann vorteilhaft vorgesehen sein, dass der schlauchförmige Abschnitt wenigstens an seinem proximalen Ende wenigstens eine Sollbruchstelle aufweist, mittels deren ein Längenabschnitt des schlauchförmigen Abschnitts abgetrennt werden kann. Der schlauchförmige Abschnitt kann beispielsweise in Umfangsrichtung umlaufende teilweise Einschnitte oder Perforationen oder sonstige Schwächungen des Materials, gegebenenfalls auch vorgegebenen durch eine molekulare Struktur, aufweisen.

Ebenso kann der schlauchförmige Abschnitt auch eine oder mehrere in axialer Richtung verlaufende Sollbruchstellen oder Reißlinien aufweisen, die an sich bei sogenannten Peel-away-Schleusen bekannt sind. Bei solchen Reißlinien kann eine Schleuse von einem Ende her durch Auseinanderziehen zweier oder mehrerer Mantelteile geöffnet und abgezogen werden. Zu diesem Zweck kann ein schlauchförmiger Abschnitt auch Handhabungselemente an seinem proximalen Ende, wie beispielsweise Ösen oder Laschen, aufweisen.

Die Schleuseneinrichtung kann gemäß der Erfindung auch eine Schneidvorrichtung aufweisen, mittels deren ein Teil des schlauchförmigen Abschnitts abgetrennt oder zur leichteren Abtrennung perforiert, eingeschnitten oder angeritzt werden kann. Beispielsweise kann eine oder können mehrere Klingen derart in das Schleusengehäuse eingesetzt werden, dass der schlauchförmige Abschnitt beim Durchschieben durch das Schleusengehäuse selbsttätig eingeschnitten wird. Ein solcher Einschnitt kann beispielsweise in Längsrichtung des schlauchförmigen Abschnitts erfolgen. Es kann auch eine Klinge vorgesehen sein, die bei einer Drehung des Schleusengehäuses relativ zu dem schlauchförmigen Abschnitt einen Schnitt oder eine Schwächung des schlauchförmigen Abschnitts in Umfangsrichtung erzeugt.

Derartige Klingen können aus einem sehr harten Material wie beispielsweise einer Keramik gefertigt sein, so dass auch verstärkte, insbesondere durch ein Metallgewebe verstärkte schlauchförmige Abschnitte geschnitten werden können. Es können auch im Wesentlichen nadelförmige Klingen vorgesehen sein, die sowohl bei einer Bewegung des schlauchförmigen Abschnitts in Axialrichtung als auch bei einer Drehung in Umfangsrichtung schneiden können.

Eine besonders vorteilhafte Ausführung der Erfindung sieht vor, dass die Schneidvorrichtung wenigstens eine Klinge aufweist, die insbesondere in dem Schleusengehäuse beweglich geführt, insbesondere radial auf den Katheter zu beweglich geführt ist. Dabei kann eine derartige Klinge mittels eines Handgriffs an der Außenseite des Schleusengehäuses betätigbar sein, so dass mit einer einfachen Handbewegung beim Herausschieben des schlauchförmigen Abschnittes aus dem Patientenkörper und dem Verschieben gegenüber dem Schleusengehäuse ein Schnitt zur Abtrennung eines Teils des schlauchförmigen Abschnitts gesetzt werden kann.

Die Erfindung bezieht sich außer auf eine Schleuseneinrichtung der oben beschriebenen Art auch auf ein Kathetersystem mit einem Katheter und einer derartigen Schleuseneinrichtung, wobei gemäß der Erfindung vorteilhaft vorgesehen sein kann, dass das Schleusengehäuse eine weitere Klemmvorrichtung auf der proximalen Seite der Klemmvorrichtung aufweist, wobei die weitere Klemmvorrichtung zum radialen Klemmen des Katheters oder zum radialen Klemmen einer zweiten Schleuse vorgesehen ist, die den Katheter und/oder ein an dem Katheter angeschlossenes Funktionselement umgibt.

Die entsprechende weitere Klemmvorrichtung kann grundsätzlich ebenfalls einen Klemmring aufweisen und beispielsweise ebenso aufgebaut sein wie die erste Klemmvorrichtung zum Fixieren des schlauchförmigen Abschnitts. Die weitere Klemmvorrichtung kann jedoch auch einen anderen Aufbau als die erste Klemmvorrichtung aufweisen und grundsätzlich gemäß einer der oben beschriebenen Varianten einer Klemmvorrichtung aufgebaut sein.

Die Erfindung bezieht sich zudem auf ein Verfahren zum Einführen eines Katheters mit einem an dessen Ende angeordnete Funktionselement in einen Patientenkörper, wobei vorgesehen ist, dass der Katheter in das Schleusengehäuse und den schlauchförmigen Abschnitt eingeführt und darauf in dem schlauchförmigen Abschnitt in den Patientenkörper eingeführt wird, dass dann der schlauchförmige Abschnitt ein Stück weit in proximaler Richtung aus dem Patientenkörper entfernt wird und dass darauf die Klemmvorrichtung gelöst und der schlauchförmige Abschnitt in das Schleusengehäuse hineinbewegt wird.

Der schlauchförmige Abschnitt kann nach dem Hineinbewegen in das Schleusengehäuse bzw. Durchschieben durch das Schleusengehäuse gekürzt werden. Vor oder nach dem Kürzen kann der schlauchförmige Abschnitt erneut mittels der Klemmvorrichtung geklemmt werden.

Vorteilhaft kann die Kürzung durch Aufreißen des schlauchförmigen Abschnitts in Längsrichtung, insbesondere vom proximalen Ende des schlauchförmigen Abschnitts her, gefolgt von einem Abreißen, erfolgen.

Im Folgenden wird die Erfindung anhand eines Ausführungsbeispiels in einer Zeichnung gezeigt und nachfolgend beschrieben.
- Fig. 1: eine schematische Übersicht über ein Gefäßsystem mit einer eingeführten ersten Schleuse;
- Fig. 2: eine Detailansicht eines Ausschnitts der Fig. 1;
- Fig. 3: eine Ausführungsform der Erfindung mit einer ersten Schleuse und einer zweiten Schleuse;
- Fig. 4: eine Ausführungsform einer Pumpe;
- Fig. 5: eine zweite Schleuse mit einer aus dieser extrahierten Pumpe;
- Fign. 6, 7: das Einziehen einer Pumpe in eine zweite Schleuse;
- Fign. 8, 9: das Überführen einer Pumpe von einer zweiten in eine erste Schleuse;
- Fig. 10: einen Längsschnitt durch ein Schleusengehäuse mit einem schlauchförmigen Abschnitt;
- Fig. 11: einen Längsschnitt durch einen Teil eines Schleusengehäuses mit einer Schneidvorrichtung;
- Fig. 12: einen Längsschnitt durch ein Schleusengehäuse mit einer Klemmvorrichtung für den schlauchförmigen Abschnitt und einer weiteren Klemmvorrichtung; und
- Fig. 13: einen Längsschnitt durch einen alternativen Klemmring mit einem konischen Druckstück.

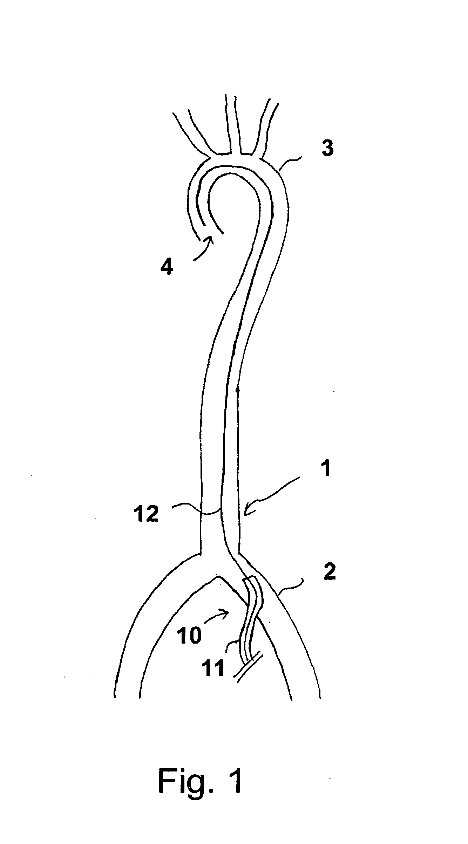

In der Fig. 1 ist ein schematisches menschliches Gefäßsystem 1 gezeigt. Im Bereich der Leiste liegt eine der femoralen Arterien 2, die über eine Hauptschlagader mit dem Aortenbogen 3 verbunden ist und anschließend in die Herzkammer 4 mündet. Mithilfe beispielsweise der Seldinger-Technik wird zunächst eine Einführschleuse 10 in die femorale Arterie 2 eingeführt. Hierbei wird zunächst die femorale Arterie bzw. ein beliebiges Blutgefäß beispielsweise mit einer Stahlkanüle mit schneidender Spitze punktiert. Durch die in die Punktur eingeführte Stahlkanüle hindurch wird ein Führungsdraht 12 geschoben und retrograd über den Aortenbogen 3 in den linken Herzventrikel 4 eingeführt. Nach Entfernung der Punktionskanüle wird die als Einführschleuse ausgebildete erste Schleuse 10, welche einen schlauchförmigen Abschnitt 11 sowie gegebenenfalls einen hier nicht dargestellten Dilatator umfasst, auf den Führungsdraht eingefädelt und durch die punktierte Stelle in das Gefäßsystem eingeführt, wobei die Schleuse eine geringe Distanz in das Lumen des Gefäßsystems oder auch bis zum Einsatzort eines einzuführenden Elementes eingeführt wird. Anschließend wird eine Fluidpumpe durch die Einführschleuse 10 in das Gefäßsystem eingeführt.

Der schlauchförmige Abschnitt 11 der ersten Schleuse 10 ist derart in die Arterie eingeführt, dass das proximale Ende der ersten Schleuse 10 außerhalb der femoralen Arterie liegt und somit zum Einführen beispielsweise einer Pumpe genutzt werden kann. So ist es möglich, die Pumpe auf dem Führungsdraht 12 aufzufädeln, um die Pumpe mithilfe des Führungsdrahtes bis in den linken Herzventrikel zu führen.

Es ist auch möglich, den schlauchförmigen Abschnitt 11 der ersten Schleuse 10 durch den Führungsdraht geführt bis in den linken Herzventrikel zu führen und den Führungsdraht 12 anschließend aus der ersten Schleuse zu entfernen. Eine etwaige Pumpeneinheit wird anschließend durch das erste Schleusenlumen bis in die Nähe oder bis in den linken Ventrikel 4 geführt.

Vorliegend wird das Verfahren lediglich anhand des Einführens einer Pumpe in den linken Herzventrikel zum Unterstützen einer Herzfunktion dargestellt. Für den Fachmann ist jedoch leicht erkennbar, dass die Pumpe oder ein anderes Funktionselement auch an andere Stellen im körpereigenen Gefäßsystem angeordnet und eingebracht werden kann.

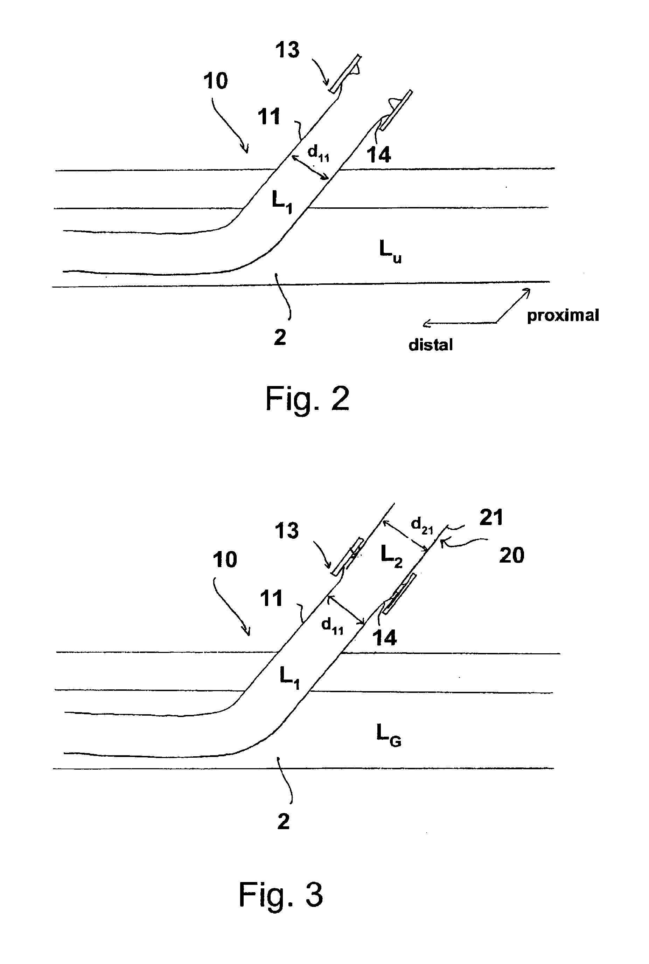

In der Fig. 2 ist der Bereich der Fig. 1 dargestellt, in welchem die erste Schleuse 10 durch das körpereigene Gewebe von außen in das Lumen L_{G} der femoralen Arterie 2 geführt ist. Die erste Schleuse umfasst dabei einen schlauchförmigen Abschnitt 11, welcher proximal mit einem Schleusengehäuse 13 verbunden ist. Der schlauchförmige Abschnitt 11 definiert ein Lumen L₁, welches einen Innendurchmesser d₁₁ aufweist. Zum proximalen Ende des schlauchförmigen Abschnitts 11 hin weitet sich dieser trompetenartig im Bereich 14 auf.

Das Schleusengehäuse 13 enthält ein im Stand der Technik bekanntes hämostatisches Ventil. Dieses verhindert, dass im Lumen L_{G} befindliches Fluid durch das Lumen L₁ nach außen austreten kann.

In der Darstellung der Fig. 3 ist die erste Schleuse 10 der Fig. 2 mit einer zweiten Schleuse 20 gekoppelt. Von der zweiten Schleuse 20 ist lediglich ein schlauchförmiger Abschnitt 21 gezeigt, welcher ein Lumen L₂ mit einem Innendurchmesser d₂₁ definiert. Das distale Ende der zweiten Schleuse 20 weist dabei einen derartigen Außendurchmesser auf, dass es in das Schleusengehäuse 13 eingeführt werden kann. Der Innendurchmesser d₂₁ ist jedoch größer als der Innendurchmesser d₁₁.

Eine nicht dargestellte, im Lumen L₂ befindliche Pumpe kann nun durch Drücken vom zweiten Schleusenlumen L₂ in das erste Schleusenlumen L₁ überführt werden. Anschließend wird die Pumpe durch das erste Schleusenlumen L₁ bis an die Stelle im Gefäßsystem transportiert, an welcher die Pumpe ihre Wirkung entfalten soll. Hierbei kann die Pumpe entweder auf einem Führungsdraht geführt werden oder ohne Führungsdraht durch das erste Schleusenlumen eingebracht werden. Die erste Schleuse kann, um die Pumpe und die Gefäßwände sowie den Wellenkatheter zu schonen, distal bis zum Einsatzort der Pumpe vorgeschoben werden, bevor die Pumpe herausgeschoben wird.

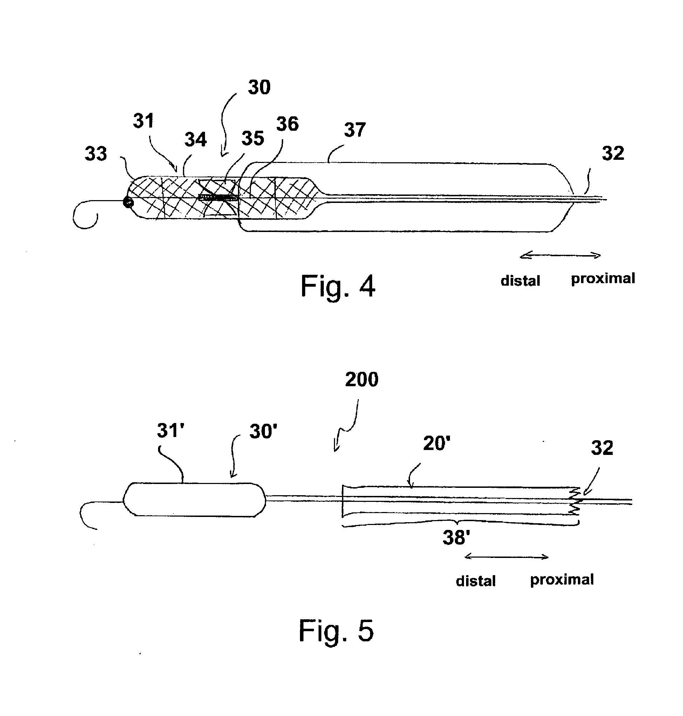

Anhand der Fig. 4 wird eine mögliche Ausführung einer Pumpe 30 genauer erläutert. Die Pumpe 30 umfasst eine distale Pumpeneinheit 31 und einen sich an das proximale Ende der distalen Pumpeneinheit 31 anschließenden Wellenkatheter 32. Der Wellenkatheter 32 weist an seinem proximalen, nicht dargestellten Ende eine Kupplung zur Ankopplung des Wellenkatheters 32 an eine Antriebsvorrichtung auf. Die Antriebsvorrichtung kann außerhalb des Patientenkörpers angeordnet sein und versetzt eine im Wellenkatheter 32 verlaufende flexible Welle in Rotation, welche wiederum die distale Pumpeneinheit 31 antreibt.

Die distale Pumpeneinheit umfasst ein Pumpengehäuse 33, welches aus sich kreuzenden Nitinolstreben hergestellt ist. Das Nitinolgehäuse ist zu Teilen mit einer Beschichtung 34 versehen, welche sich distal und proximal eines im Gehäuse 33 angeordneten Rotors 35 erstreckt. Der Rotor ist mit der durch den Wellenkatheter 32 verlaufenden Welle 36 verbunden und wird so in Drehung versetzt. Das Gehäuse und der Rotor sind komprimierbar, d. h., die Pumpe ist eine selbstentkomprimierbare Pumpe. Die Entfaltung der Pumpe vollzieht sich nach dem Herausschieben der distalen Pumpeneinheit aus dem distalen Ende einer Schleuse. Zum Komprimieren der Pumpe in Vorbereitung der Implantation wird die distale Pumpeneinheit in das distale Ende eines Schleusenlumens einer zweiten Schleuse eingezogen. Das Schleusenlumen besitzt dabei einen Innendurchmesser, welcher zumindest größer als der Außendurchmesser des Wellenkatheters ist.

Der Rotor kann gegenüber dem Pumpengehäuse in Axialrichtung verschiebbar sein, insbesondere mittels einer Axialverschiebung der Antriebswelle. Der Rotor kann andererseits auch in Axialrichtung gegenüber dem Pumpengehäuse fixiert sein.

Optional weist die Pumpe einen Abströmschlauch 37 auf, welcher einen proximal des Rotors 35 gelegenen Strömungskanal für das gepumpte Fluid definiert. Am proximalen Ende des Abströmschlauchs 37 befinden sich nicht näher dargestellte Auslassöffnungen.

Selbstverständlich kann die Pumpe auch von einem Pumpbetrieb in einen Saugbetrieb umgeschaltet werden, so dass die Pumpe nicht länger Fluid vom distalen Ende ans proximale Ende führt, sondern umgekehrt.

Eine detailliertere Beschreibung einer weiteren geeigneten Pumpe kann beispielsweise der Druckschrift EP 2 047 872 A1 entnommen werden.

Anhand der Figuren 5 bis 9 soll nun die Funktion des Systems erläutert werden.

In der Fig. 5 ist eine Pumpe 30' dargestellt, welche im Wesentlichen der Pumpe 30 nach der Fig. 4 entspricht. Zur Vereinfachung sind Details der Pumpe nicht gezeigt. Es sind lediglich das bauchige Gehäuse sowie das distal des bauchigen Gehäuses gelegene "Pigtail" dargestellt, welches ein Ansaugen der Herzpumpe an die Herzwand verhindert. Proximal der distalen Pumpeneinheit 31' verläuft der Wellenkatheter 32'. Einen Bereich 38' des Wellenkatheters 32' umschließend ist eine zweite Schleuse 20' angeordnet, welche ein Lumen L₂ umfasst, dessen Innendurchmesser d₂₁ kleiner als der Durchmesser der distalen Pumpeneinheit 31' im entfalteten Zustand ist.

Die in der Fig. 5 dargestellte Pumpe 30' ist eine komprimierbare Pumpe, das heißt, die distale Pumpeneinheit 31', welche unter anderem das Pumpengehäuse und den darin befindlichen Rotor umfasst, ist derart ausgebildet, dass sie komprimiert, d. h. in ihrem Durchmesser verringert werden kann. Nachdem ein Qualitätsprüfer oder beispielsweise ein Arzt sich von der korrekten Funktion der Pumpe 30' überzeugen konnte, z. B. durch Betrachtung der Rotationsbewegung der in der distalen Pumpeneinheit 31' befindlichen Rotoreinheit bei einem Testlauf, wird durch Ziehen des Wellenkatheters 32' in proximaler Richtung die distale Pumpeneinheit 31' in das Lumen L₂ der zweiten Schleuse 20' gezogen. Durch das Einziehen der Pumpe in die zweite Schleuse 20' wird ein Verbiegen bzw. eine Beschädigung des Wellenkatheters bzw. der darin verlaufenden Welle vermieden. Die in der Fig. 5 dargestellte Pumpe 30' und die den Bereich 38' des Wellenkatheters 32' umschließende zweite Schleuse 20' bilden ein System 200, welches es ermöglicht, rechtzeitig vor einer Operation die Funktion der Pumpe 30' zu testen und anschließend durch das Ziehen der distalen Pumpeneinheit 31' in das distale Ende der zweiten Schleuse 20' die Pumpe zu komprimieren und dabei eine Beschädigung der Welle zu vermeiden.

Obgleich das System sowohl mit aktiv entkomprimierbaren Pumpen als auch mit selbstentkomprimierbaren Pumpen realisierbar ist, eignet es sich insbesondere für selbstentkomprimierbare Pumpen, d.h. Pumpen, deren distale Pumpeneinheit außerhalb der Schleuse selbsttätig wieder die ursprüngliche Größe annimmt.

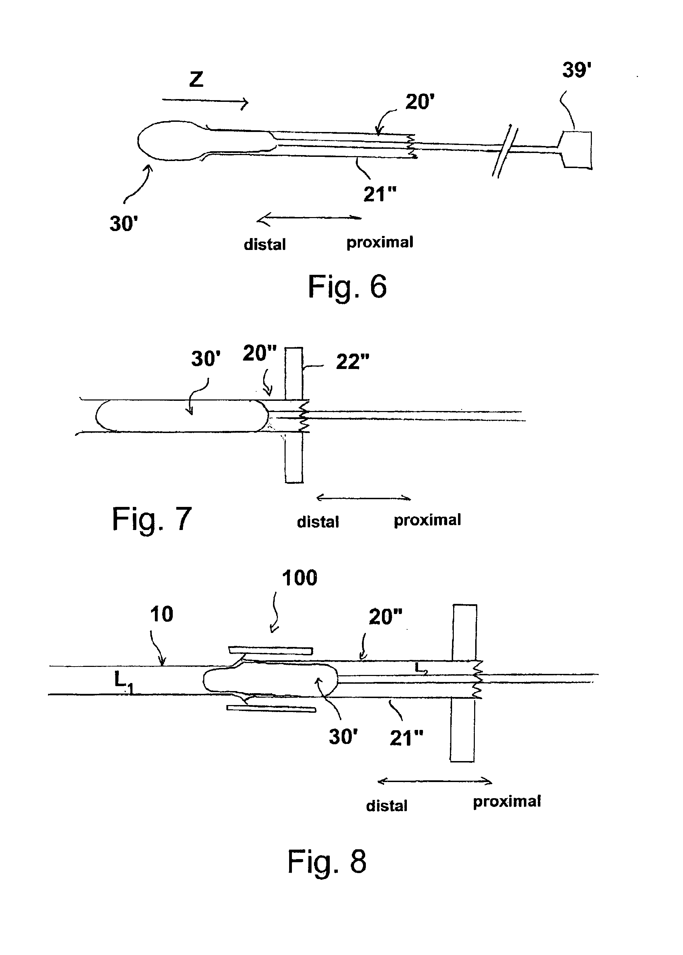

In der Fig. 6 ist ein Zwischenschritt beim Einziehen der distalen Pumpeneinheit 30' in das Lumen der zweiten Schleuse 20' dargestellt. Es ist erkennbar, dass die distale Pumpeneinheit 30' komprimierbar ist und auf einen geringeren Durchmesser gebracht werden kann, so dass die distale Pumpeneinheit 30' in das Lumen der zweiten Schleuse 20' aufgenommen werden kann.

Ferner ist in der Fig. 6 eine an den Wellenkatheter 32' anschließende Kupplung 39' dargestellt, welche ein Ankoppeln der in dem Wellenkatheter verlaufenden Welle an eine Antriebseinheit ermöglicht. Da die Kupplung 39' einen oftmals größeren Außendurchmesser als den Innendurchmesser des Lumens L₂ aufweist, wird die zweite Schleuse 20' zumeist vor der Montage der Kupplung 39' vom proximalen Ende des Wellenkatheters 32' her in distaler Richtung aufgesetzt, so dass die Pumpe im System 200, d. h. die Pumpe mit der sich proximal der distalen Pumpeneinheit 31' befindlichen zweiten Schleuse 20' und der vormontierten Kupplung 39', ausgeliefert wird. An der Fig. 6 ist auch eine leichte trompetenförmige Aufweitung des distalen Endes der zweiten Schleuse 20' dargestellt. Die trompetenartige Aufweitung 24' erleichtert das Einziehen der distalen Pumpeneinheit 31' in das Lumen L₂ der zweiten Schleuse 20'.

In der Fig. 7 schließlich befindet sich die distale Pumpeneinheit 31' vollständig im Lumen L₂ der zweiten Schleuse 20". Die zweite Schleuse 20" weist zwei vormontierte Griffeinheiten 22" auf, welche ein besseres Halten bzw. Entfernen der zweiten Schleuse 20" beim Einziehen der distalen Pumpeneinheit 31' in das Lumen L₂ bzw. ein anschließendes Aufreißen ermöglichen. Vorteilhafterweise wird bei vorhandenem "Pigtail" dieses ebenfalls in das Lumen L₂ eingezogen, so dass sich die distale Pumpeneinheit 31' samt distal der distalen Pumpeneinheit 31' gelegenen Komponenten der Pumpe im Lumen L₂ befindet.

In der Fig. 8 wird erkennbar, wie das System 200 aus Pumpe 30' und zweiter Schleuse 20" in Wirkverbindung mit der ersten Schleuse 10 zu einem System 100 kombiniert wird. Zunächst wird die zweite Schleuse 20" mit ihrem distalen Ende in das Schleusengehäuse der ersten Schleuse 10 eingeführt. Sobald die distale Spitze der zweiten Schleuse 20" an der Mündung des schlauchförmigen Abschnitts der ersten Schleuse 10 anliegt, wird durch Schieben der Pumpe in distaler Richtung, wobei das Schieben anhand eines Schiebens des Wellenkatheters 32' erfolgt, die Pumpe von der zweiten Schleuse 20' in die erste Schleuse 10' überführt. Hierbei wird der Durchmesser der distalen Pumpeneinheit 31' weiter auf den Innendurchmesser d₁₁ des Lumens L₁ reduziert.

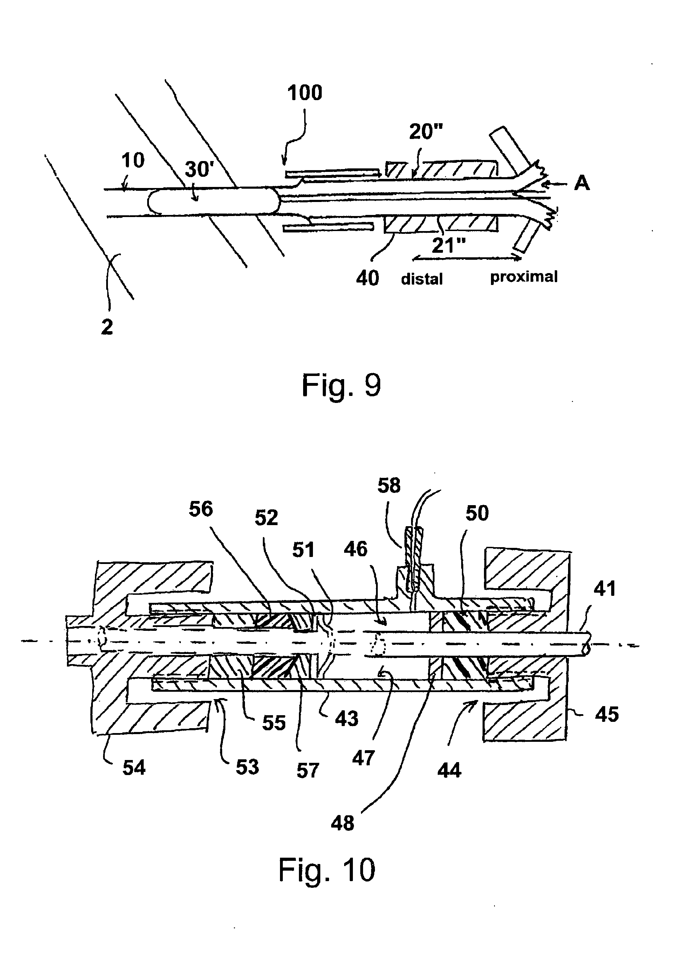

In der Fig. 9 ist der anschließende Schritt dargestellt, bei welchem sich die distale Pumpeneinheit 31' vollständig im Lumen L₁ der ersten Schleuse 10 befindet. Dass sich die distale Pumpeneinheit 31' vollständig im Lumen L₁ der ersten Schleuse 10 befindet, kann beispielsweise anhand einer farblichen Markierung 50, welche auf der Außenseite des Wellenkatheters 32' aufgebracht ist, kenntlich gemacht werden.

Anschließend wird die zweite Schleuse 20", welche als eine "Peel-away"-Schleuse ausgebildet ist, vom Wellenkatheter 32' entfernt, indem die Peel-away-Schleuse vom proximalen zum distalen Ende hin aufgerissen und vom Wellenkatheter 32' abgezogen wird. Das gerichtete Aufreißen vom proximalen zum distalen Ende hin kann durch Einkerbungen A unterstützt werden, beruht jedoch vorwiegend auf der Ausrichtung der Molekülketten des verwendeten Kunststoffs von der proximalen in die distale Richtung.

Nachdem die Peel-away-Schleuse entfernt wurde, wird die Pumpe 30' weiter innerhalb des Lumens L₁ der ersten Schleuse 10 bis zum gewünschten Ort geführt.

Wahlweise kann die erste Schleuse auch vor oder nach dem Einführen der Pumpe mit der distalen Schleusenmündung in die unmittelbare Nähe des Einsatzortes vorgeschoben werden. Hierzu weist die erste Schleuse die notwendige Länge auf.

Eine Versteifung der zweiten Schleuse 20" ist insbesondere beim Einziehen der distalen Pumpeneinheit 31' in das distale Ende des zweiten Schleusenlumens L₂ nicht notwendig, da die Gefahr eines Abknickens der Welle bei einer Ziehbewegung stark reduziert ist.

Beim Überführen der Pumpe von der zweiten Schleuse in die erste Schleuse, wie anhand der Figuren 7 bis 9 dargestellt, kann die zweite Schleuse eine sie verstärkende Struktur in Form eines eingebrachten Drahtes umfassen, oder der schlauchförmige Abschnitt 21" der Schleuse 20" wird nicht aus einem flexiblen Kunststoff, sondern aus einem formfesten Kunststoff oder Metall hergestellt.

Eine weitere Möglichkeit zum Stabilisieren der Pumpe und der zweiten Schleuse besteht darin, beim Vorschieben der Pumpe 30' in distaler Richtung, d.h. insbesondere beim Überführen der Pumpe 30' von der zweiten Schleuse in die erste Schleuse, die zweite Schleuse 20" durch eine Stützvorrichtung 40 in Form einer stabilen äußeren Hülse zu halten.

Anschließend soll noch eine mögliche Variante eines Verfahrens zum Einführen einer Pumpe in einen linken Herzventrikel geschildert werden. Als vorbereitende Maßnahme wird die Pumpe zunächst mit steriler physiologischer Kochsalzlösung befüllt und somit komplett entlüftet. Anschließend wird die proximal der distalen Pumpeneinheit gelegene Peel-away-Schleuse bis zu einem eventuell vorhandenen Abströmschlauch vorgeschoben. Die Peel-away-Schleuse besitzt einen Durchmesser von beispielsweise 10 Fr. Nachdem die Peel-away-Schleuse bis zum Abströmschlauch vorgeschoben wurde, wird die Peel-away-Schleuse von einer hülsenförmigen Vorrichtung zum Halten der zweiten Schleuse umfasst. Anschließend wird die distale Pumpeneinheit, gegebenenfalls unter leichter Drehbewegung, in die Peel-away-Schleuse eingezogen, indem am Wellenkatheter eine Zugbewegung in proximaler Richtung ausgeübt wird. Die Pumpe wird so weit in die zweite Schleuse verschoben, dass ein eventuell vorhandenes Pigtail ebenfalls in der Peel-away-Schleuse geborgen ist. Durch diese Schritte ist es möglich, die Funktionsfähigkeit der Pumpe bereits vor einem operativen Eingriff zu überprüfen und die Pumpe erst anschließend in eine Schleuse einzuführen, ohne unter Zeitdruck agieren zu müssen. Zum Beispiel erst anschließend wird die Punktur des Gefäßsystems zum Einführen der ersten Schleuse durchgeführt. Zur Zeiteinsparung ist es auf diese Weise aber auch möglich, dass ein Assistent die Pumpe vorbereitet während der Anwender bereits parallel die Punktion durchführt.

Nachdem beispielsweise eine 9Fr-Einführschleuse bis in den linken Herzventrikel eingeführt wurde, wird ein eventuell vorhandener Dilatator aus der Einführschleuse gezogen und aus dieser entfernt.

Anschließend wird die in der Peel-away-Schleuse gehaltene Pumpe, welche beispielsweise anfangs von der Hülse zum Halten der zweiten Schleuse umfasst wird, in das Schleusengehäuse eingeschoben, bis die Spitze der Peel-away-Schleuse an einem mechanischen Anschlag anschlägt. Anschließend wird die Pumpe durch Schieben des Wellenkatheters von der Peel-away-Schleuse in den schlauchförmigen Abschnitt überführt. Sobald die distale Pumpeneinheit komplett in die Einführschleuse überführt wurde, wie dies beispielsweise anhand einer optischen Markierung auf dem Wellenkatheterschaft überprüfbar ist, kann die Peel-away-Schleuse aufgerissen und vom Wellenkatheter abgezogen werden. Anschließend wird die Pumpe innerhalb der ersten Schleuse bis in den linken Herzventrikel vorgeschoben. Die erste Schleuse wird anschließend aus dem linken Herzventrikel bis zum Beginn der absteigenden Aorta zurückgezogen.

Die Positionierung der distalen Pumpeneinheit im linken Herzventrikel kann beispielsweise durch Röntgendurchleuchtung kontrolliert werden. Hierzu befindet sich eine röntgensichtbare Markierung am Pumpengehäuse bzw. in dessen Nähe, beispielsweise am Katheter, oder das Pumpengehäuse selbst ist röntgensichtbar. Ebenso sollte der Auslassbereich der Pumpe, d.h. die Ausströmöffnungen eines Abströmschlauches, im Bereich der aufsteigenden Aorta liegen. Auch dies kann mit einer röntgensichtbaren Markierung überprüft werden. Eine eventuell vorhandene Pigtail-Katheterspitze sollte an der Spitze des linken Herzventrikels anstoßen.

Um die Pumpe aus dem Herzventrikel zu entfernen, wird diese mittels am Wellenkatheter aufgebrachter Zugkraft in die Einführschleuse zurückgezogen und komprimiert aus dem arteriellen Gefäßsystem entfernt. Sie kann auch, wenn die erste Schleuse bereits gekürzt ist, zunächst in den Wellenkatheter ein Stück weit zurückgezogen werden, um die Pumpe zu komprimieren. Hierzu kann der Wellenkatheter einen Einzugtrichter aufweisen, in den die Pumpe durch Zug an der Antriebswelle eingezogen werden kann. Anschließend werden die erste Schleuse und weitere verbliebene Komponenten aus dem Gefäßsystem entfernt.

Besonderen Vorteil bringt bei der Erfindung die Verwendung einer langen Schleuse bei der Implantation und Explantation der Pumpe. Die lange Schleuse dient nicht nur, wie im Stand der Technik üblich, zum Einführen der Pumpe in ein körpereigenes Lumen, sondern zur Führung der Pumpe durch das Schleusenlumen in die Nähe des Wirkungsorts. Hierbei ist es vorteilhaft, wenn im medizinischen Bereich die Schleuse eine Länge zwischen 40 und 120 cm besitzt. Die Länge wird durch den späteren Wirkungsort der Pumpe und die Physis des Patienten bestimmt.

Wird die Pumpe zusammen mit der langen Schleuse aus einem körpereigenen Lumen herausgezogen, so wird die Blutung der femoralen Arterie mit einem Druckverband gestillt. Alternativ kann die Pumpe aus dem Schleusenlumen der langen Schleuse herausgezogen werden. Dann kann durch das Lumen der Schleuse ein weiterer Führungsdraht platziert werden, über den dann nach Entfernung der Schleuse eine Vorrichtung zum Schließen der Punktur geführt werden kann. Hierdurch ist eine verbesserte Blutungsstillung erreichbar.

Die Figuren 10 bis 13 zeigen speziell eine erfindungsgemäße Ausführung der ersten Schleuse mit einer bzw. mehreren Klemmvorrichtungen zur Fixierung eines schlauchförmigen Abschnittes 41 in einem Schleusengehäuse 43.

Fig. 10 zeigt dazu in einem Längsschnitt ein Schleusengehäuse 43, das im Wesentlichen die Form einer zylindrischen Hülse aufweist, die zumindest an dem distalen, dem Patientenkörper zugewandten Ende 44 durch eine Druckschraube 45 abgeschlossen ist. Das Schleusengehäuse 43 weist einen durchgehenden Aufnahmekanal 46 für einen schlauchförmigen Abschnitt 41 der ersten Schleuse auf. In der Darstellung der Fig. 10 ist der schlauchförmige Abschnitt 41 vom Patientenkörper kommend bis in den Spülraum 47 des Aufnahmekanals 46 durchgehend, anschließend in proximaler Richtung gestrichelt dargestellt. Dies deutet an, dass der schlauchförmige Abschnitt 41 gegenüber dem Schleusengehäuse 43 innerhalb des Aufnahmekanals 46 axial verschiebbar ist oder, anders ausgedrückt, dass das Schleusengehäuse 43 auf dem schlauchförmigen Abschnitt 41 verschiebbar ist.

Zum Einführen eines Funktionselementes, beispielsweise einer Pumpe, in die erste Schleuse wird üblicherweise der schlauchförmige Abschnitt 41 so weit in distaler Richtung aus dem Schleusengehäuse 43 herausgezogen oder bei der Herstellung der ersten Schleuse so positioniert, dass er etwa in Höhe des ersten Anschlagstückes 48 endet. Bis zu diesem Punkt kann dann eine zweite Schleuse mit einer eingezogenen Pumpe, wie oben beschrieben, vorgeschoben werden, um dann die Pumpe von der zweiten Schleuse in die erste Schleuse durchzubewegen.

Die erste Klemmvorrichtung weist als Elemente die erste Druckschraube 45, einen ersten Klemmring 50 aus einem Elastomermaterial sowie das erste Anschlagstück 48 auf.

Die Druckschraube ist mittels eines Außengewindes im Bereich der Überlappung mit dem distalen Ende 44 des Schleusengehäuses 43 mit diesem verschraubt. Ein manuelles Drehen an der Druckschraube 45 bewirkt damit eine Bewegung der Druckschraube in axialer Richtung, die zu einer axialen Kompression oder Ausdehnung des Klemmrings 50 führt. Bei einer axialen Kompression hat der Klemmring 50 die Tendenz, zur Erhaltung seines Volumens radial nach innen und außen auszuweichen, und klemmt damit den schlauchförmigen Abschnitt 41, da er auf seiner proximalen Seite einen Widerstand durch das erste Anschlagstück 48 erfährt.

Damit wird der schlauchförmige Abschnitt 41 axial gegenüber dem Schleusengehäuse 43 fixiert. Diese Fixierung kann einfach durch Lösen der Druckschraube 45 aufgelöst werden, so dass der schlauchförmige Abschnitt 41 dann leicht axial in dem Schleusengehäuse 43 verschoben werden kann. Der Klemmring kann dazu im entspannten Zustand einen Innendurchmesser aufweisen, der gleich wie oder größer als der Durchmesser der ersten Schleuse ist.

Wird also zunächst der schlauchförmige Abschnitt 41 möglichst weit in den Patientenkörper hineingeschoben, um ein Einführen der Pumpe in dem Schutz der Schleuse bis zum Einsatzort, beispielsweise in einem Herzventrikel, zu ermöglichen, so wird nach dem Ausbringen der Pumpe der schlauchförmige Abschnitt 41 herausgezogen, und die Schleuse insgesamt steht relativ weit aus dem Patientenkörper vor. Danach kann die Klemmvorrichtung 48, 45, 50 gelöst und das Schleusengehäuse 43 auf dem schlauchförmigen Abschnitt 41 näher zum Patientenkörper herangeschoben werden. Dabei durchsetzt der schlauchförmige Abschnitt 41 das Schleusengehäuse 43 dann vollständig und ragt gegebenenfalls in proximaler Richtung aus diesem heraus. Darauf kann mit Mitteln, die weiter unten ausführlicher beschrieben werden, der schlauchförmige Abschnitt 41 teilweise abgetrennt werden, um die Überlänge zu entfernen.

Innerhalb des Schleusengehäuses 43 ist zur besseren Abdichtung ein sogenanntes kombiniertes Hämostaseventil, bestehend aus einem Domventil 51 und einer Ventilplatte 52 vorgesehen. Die Ventilplatte schließt das Schleusengehäuse 43, wenn an dieser Stelle weder der schlauchförmige Abschnitt 41 noch ein Wellenkatheter den Aufnahmekanal 46 durchsetzt, während das Domventil 51 für den dichten Abschluss um einen strangförmigen Körper herum, beispielsweise den schlauchförmigen Abschnitt oder einen Katheter, optimiert ist.

Am proximalen Ende 53 des Schleusengehäuses 43 ist eine weitere Druckschraube 54 vorgesehen, die im Prinzip ebenso funktioniert wie die erste Druckschraube 45 und die über ein Druckstück 55 die Kompression eines zweiten Klemmrings 56 gegenüber einem zweiten mechanischen Anschlag 57 bewirkt. Als Besonderheit ist hier zu erwähnen, dass der zweite Klemmring 56 an seinem distalen Ende konisch zuläuft, was eine Verformung radial nach innen beim Ausüben eines axialen Drucks durch die Druckschraube 54 begünstigt. Entsprechend gegensinnig ist der zweite Anschlag 57 konisch ausgebildet. Es kann jedoch an dieser Stelle ebenso ein nicht konischer, sondern im Querschnitt rechteckiger oder runder Klemmring 56 eingesetzt werden.

Es kann/können auch zusätzlich im Spülraum ein oder mehrere weitere(s) Ventil(e) zwischen der Klemmvorrichtung 48, 45,50 und dem Spüleinlass 58 angeordnet werden, wodurch sichergestellt wird, dass zwischen dem schlauchförmigen Abschnitt 41 und dem Schleusengehäuse 43, auch bei gelöster Klemmvorrichtung 48,45, 50 eine fluiddichte Verbindung besteht.

In der Fig. 10 ist eine Spülvorrichtung 58 schematisch angedeutet, die das Spülen des Spülraums 47 mit einer Flüssigkeit ermöglicht, die das Eindringen von Keimen durch die erste Schleuse in den Patientenkörper verhindert. Diese Spülung ist besonders dann effektiv, wenn der schlauchförmige Abschnitt 41 in dem Spülraum 47 oder auf dessen distaler Seite endet, so dass sowohl die Außenseite als auch die Innenseite des schlauchförmigen Abschnitts 41 von der Spülflüssigkeit erreicht werden.

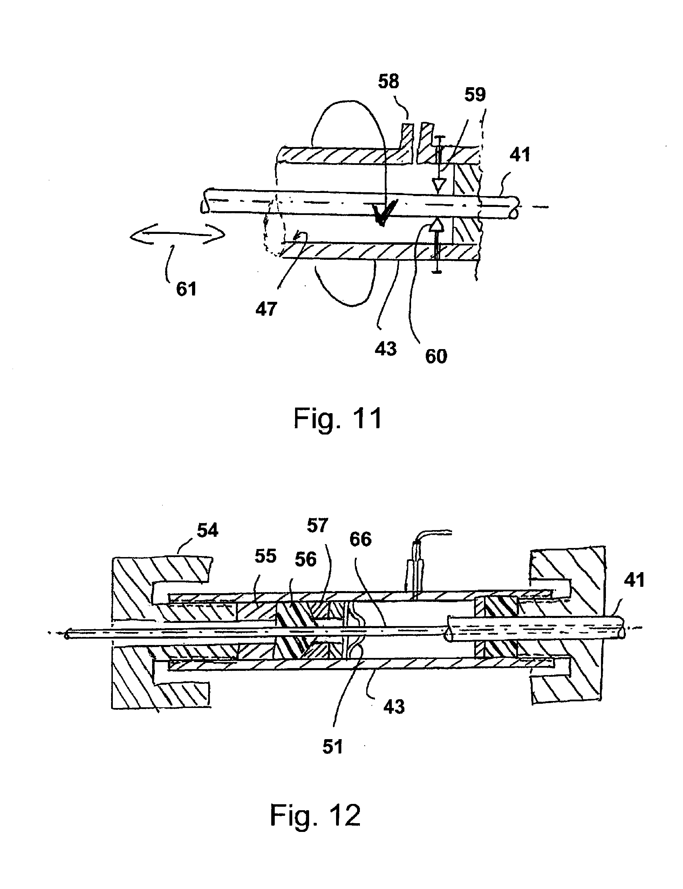

Fig. 11 zeigt exemplarisch die Anordnung und Funktionsweise einer erfindungsgemäßen Schneidvorrichtung.

Wenn keine vorgeschnittenen oder in anderer Weise, beispielsweise durch eine vorgegebene Molekülstruktur oder stellenweise Schwächung der Wandstärke des schlauchförmigen Abschnitts 21, vorgegebenen Sollbruchstellen vorgesehen sind, können diese passend bei Verwendung der ersten Schleuse durch eine Schneidvorrichtung eingebracht werden. In der Fig. 11 ist im Bereich des Spülraumes 47 des Schleusengehäuses 43 eine Schneidvorrichtung mit Klingen 59, 60 vorgesehen, die beispielsweise bei einer Drehung des Schleusengehäuses gegenüber dem schlauchförmigen Abschnitt diesen in Umfangsrichtung schneiden. Es können auch Schnitte in Axialrichtung eingebracht werden.

Zu diesem Zweck können die Klingen 59, 60 auch so angeordnet sein, dass sie bei einer Bewegung des schlauchförmigen Abschnitts 41 in axialer Richtung, wie durch den Pfeil 61 angedeutet, in Längsrichtung schneiden. Es können auch sowohl Klingen für ein Schneiden in Umfangsrichtung als auch für ein Schneiden in Längsrichtung vorgesehen sein.

In der Fig. 11 ist außerdem dargestellt, dass die Klingen 59, 60 in Richtung radial auf den schlauchförmigen Abschnitt 41 zu durch eine Betätigung von der Außenseite des Schleusengehäuses 43 bewegt werden können. Dort können eine in Radialrichtung verlaufende Führung für einen oder mehrere Klingenhalter, eine entsprechende Dichtung und eine Federung vorgesehen sein, so dass das Eindringen von Keimen durch diese Verschiebungsvorrichtung für die Klingen verhindert wird und die Klingen im nicht betätigten Zustand radial einen Abstand zu dem schlauchförmigen Abschnitt 41 einnehmen. Nach der Verwendung der ersten Schleuse kann dann von Hand ein Druck auf die Klingen ausgeübt und der nicht benötigte Teil des schlauchförmigen Abschnitts 41 abgeschnitten werden. Ein hier nicht dargestellter Anschlag verhindert, dass die Schnitttiefe ein kritisches Maß übersteigt und dadurch ein eventuell innerhalb der Schleuse befindlicher Katheter beschädigt wird.

Die dargestellten Klingen können auch eine Schneidvorrichtung für eine zweite Schleuse bilden.

Die Fig. 12 zeigt eine vorteilhafte Verwendung der zweiten Klemmvorrichtung auf der proximalen Seite des Schleusengehäuses 43, nachdem der schlauchförmige Abschnitt 41 gekürzt ist und ein Wellenkatheter 61 aus dem proximalen Ende des schlauchförmigen Abschnitts 41 heraus und weiter zu einer nicht dargestellten Kopplungsvorrichtung für eine antreibbare Welle einer Pumpe aus dem Schleusengehäuse 43 führt. Der Wellenkatheter ist in der oben angesprochenen Domdichtung 51 gedichtet, und die Klemmvorrichtung mit den Elementen der zweiten Druckschraube 54 und dem zweiten Klemmring 56, der durch das Druckstück 55 gegenüber einem zweiten Anschlag 57 axial komprimiert ist, weicht so weit radial nach innen aus, dass er den Wellenkatheter 61, der einen wesentlich geringeren Außendurchmesser aufweist als der schlauchförmige Abschnitt 41 oder eine zweite Schleuse, klemmt und insbesondere auch zusätzlich dichtet. Auf diese Weise können in dem Schleusengehäuse 43 sowohl der schlauchförmige Abschnitt 41 als auch der aus diesem herausragende Wellenkatheter 61 fixiert werden.

Die zweite Klemmvorrichtung ist ebenso dazu geeignet, beim Einführen einer zweiten Schleuse in das Schleusengehäuse 43 die zweite Schleuse mit dem zweiten Klemmring 56 so zu fixieren, dass sie gegenüber dem Schleusengehäuse 43 und insbesondere auch gegenüber dem schlauchförmigen Abschnitt 41 zum Durchschieben des Wellenkatheters 61 ausreichend fixiert ist.

Der erste und zweite Klemmring 50, 56 können aus einem Elastomer, beispielsweise einem Gummi oder einem Silikonelastomer, bestehen und somit vollelastisch, aber volumeninkompressibel verformbar sein. Es ist an dieser Stelle jedoch auch die Verwendung eines elastischen Schaumstoffs denkbar, der teilweise volumenkompressibel ist.

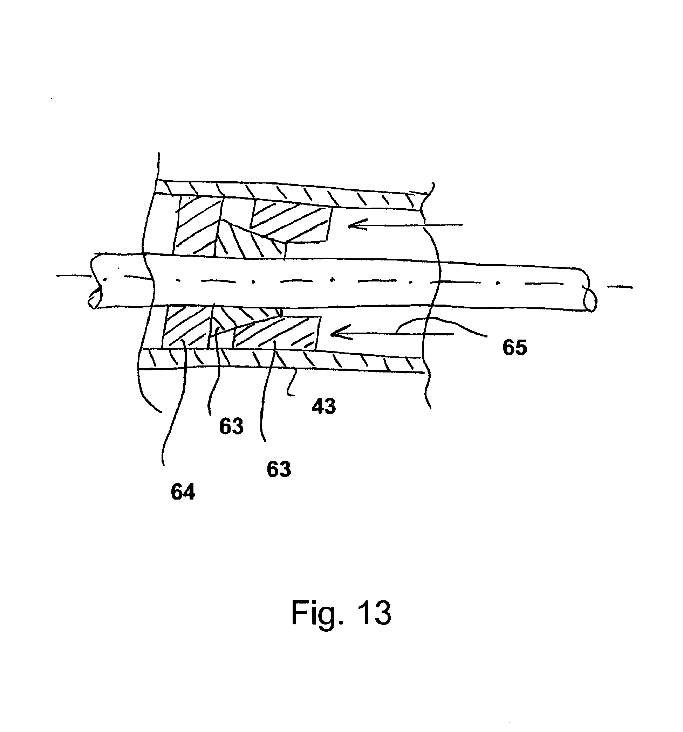

In der Fig. 13 ist schematisch eine andere Art eines Klemmrings 62 gezeigt, der beispielsweise aus einem Kunststoff oder einem Metall bestehen kann und insbesondere geschlitzt und damit radial komprimierbar ist. Der geschlitzte Klemmring 62 weist eine konische Außenkontur auf, gegen die die konische Kontur eines Druckstücks 63 drückt, um den Klemmring radial zu komprimieren, sobald auf das Druckstück 63 eine axiale Druckkraft in Richtung des Pfeils 65, beispielsweise durch eine oben dargestellte Druckschraube, ausgeübt wird. Der geschlitzte Klemmring 62 ist axial durch das Anschlagstück 64 fixiert.

## Patentansprüche

1. Schleuseneinrichtung zum Einführen eines Katheters in einen Patientenkörper mit einer ersten Schleuse (10, 11, 13, 21, 21', 21", 41, 43) mit einem proximalen Ende und einem distalen Ende, wobei beim bestimmungsgemäßen Gebrauch das distale Ende der ersten Schleuse zur Anordnung im Patientenkörper und das proximale Ende der ersten Schleuse zur Anordnung außerhalb des Patientenkörpers vorgesehen ist und wobei die erste Schleuse einen schlauchförmigen Abschnitt (11, 21, 21', 21", 41) und ein an dessen proximalem Ende angeordnetes Schleusengehäuse (13, 43) mit einem Aufnahmekanal (46) für einen strangförmigen Körper, insbesondere für einen Katheter aufweist und wobei der schlauchförmige Abschnitt (11, 21, 21', 21", 41) in einer Klemmvorrichtung (45, 48, 50, 62, 63, 64) des Schleusengehäuses lösbar kraftschlüssig gehalten ist.

2. Schleuseneinrichtung nach Anspruch 1, **dadurch gekennzeichnet, dass** der schlauchförmige Abschnitt (11, 21, 21', 21", 41) im gelösten Zustand der Klemmvorrichtung (45, 48, 50, 62, 63, 64) in das Schleusengehäuse hinein verschiebbar ist.

3. Schleuseneinrichtung nach Anspruch 1 oder 2, **dadurch gekennzeichnet, dass** der schlauchförmige Abschnitt (11, 21, 21', 21", 41) in direkter Verlängerung des Aufnahmekanals (46) in dem Schleusengehäuse mündet.

4. Schleuseneinrichtung nach Anspruch 1, 2 oder 3, **dadurch gekennzeichnet, dass** die Klemmvorrichtung einen elastisch verformbaren Klemmring (50, 62) aufweist, der den schlauchförmigen Abschnitt (11, 21, 21', 21", 41) umgibt und der durch eine Manipulationsvorrichtung derart pressbar ist, dass er den schlauchförmigen Abschnitt radial einklemmt.

5. Schleuseneinrichtung nach Anspruch 4, **dadurch gekennzeichnet, dass** der Klemmring (50, 62) durch axiale Druckwirkung radial verformbar ist.

6. Schleuseneinrichtung nach Anspruch 1, 2, 3, 4 oder 5, **dadurch gekennzeichnet, dass** der Klemmring (50) aus einem Elastomer oder aus einem geschlitzten Ring (62) aus Kunststoff oder Metall besteht.

7. Schleuseneinrichtung nach Anspruch 4 oder einem der folgenden, **dadurch gekennzeichnet, dass** die Klemmvorrichtung eine Schraubvorrichtung (43, 45) zur axialen Pressung eines Klemmringes (50, 62) aufweist.

8. Schleuseneinrichtung nach Anspruch 1 oder einem der folgenden, **dadurch gekennzeichnet, dass** der schlauchförmige Abschnitt (11, 21, 21', 21", 41) wenigstens an seinem proximalen Ende wenigstens eine Sollbruchstelle aufweist, mittels deren ein Längenabschnitt des schlauchförmigen Abschnitts abgetrennt werden kann.

9. Schleuseneinrichtung nach Anspruch 1 oder einem der folgenden, **dadurch gekennzeichnet, dass** das Schleusengehäuse (13, 43) eine Schneidvorrichtung (59, 60) aufweist, mittels deren ein Teil des schlauchförmigen Abschnitts (11, 21, 21', 21", 41) abgetrennt oder zur leichteren Abtrennung perforiert, eingeschnitten oder angeritzt werden kann.

10. Schleuseneinrichtung nach Anspruch 9, **dadurch gekennzeichnet, dass** die Schneidvorrichtung wenigstens eine Klinge (59, 60) aufweist, die insbesondere in dem Schleusengehäuse (13, 43) beweglich geführt, insbesondere radial auf den Katheter zu beweglich geführt ist.

11. Kathetersystem mit einem Katheter und einer Schleuseneinrichtung nach Anspruch 1 oder einem der folgenden, **dadurch gekennzeichnet, dass** das Schleusengehäuse (13, 43) eine weitere Klemmvorrichtung (54, 55, 56, 57) auf der proximalen Seite der Klemmvorrichtung (45, 48, 50, 62, 63, 64) aufweist, wobei die weitere Klemmvorrichtung zum radialen Klemmen des Katheters (66) oder zum radialen Klemmen einer zweiten Schleuse vorgesehen ist, die den Katheter und/oder ein an dem Katheter angeschlossenes Funktionselement umgibt.

12. Verfahren zum Einführen eines Katheters mit einem an dessen Ende angeordneten Funktionselement in einen Patientenkörper, **dadurch gekennzeichnet, dass** der Katheter in das Schleusengehäuse (13, 43) und den schlauchförmigen Abschnitt (11, 21, 21', 21", 41) eingeführt und darauf in dem schlauchförmigen Abschnitt in den Patientenkörper eingeführt wird, dass dann der schlauchförmige Abschnitt ein Stück weit in proximaler Richtung aus dem Patientenkörper entfernt wird und dass darauf die Klemmvorrichtung (45, 48, 50, 62, 63, 64) gelöst und der schlauchförmige Abschnitt in das Schleusengehäuse hineinbewegt wird.

13. Verfahren nach Anspruch 12, **dadurch gekennzeichnet, dass** der schlauchförmige Abschnitt (11, 21, 21', 21", 41) nach dem Einziehen in das Schleusengehäuse (13, 43) gekürzt und erneut geklemmt wird.

14. Verfahren nach Anspruch 13, **dadurch gekennzeichnet, dass** die Kürzung durch ein Aufreißen des schlauchförmigen Abschnittes (11, 21, 21', 21", 41) in Längsrichtung, gefolgt von einem Abreißen, erfolgt.
